# EUROPEAN PATENT SPECIFICATION

(11) **EP 2 538 906 B1**
(45) Date of publication and mention of the grant of the patent: **27.03.2019**
(21) Application number: 11748238.0
(22) Date of filing: 28.02.2011
(51) Int. Cl.: A61F 13/38, G01N 1/02, A61F 15/00, A61B 10/02, A61B 10/00

(54) **EVIDENCE COLLECTOR WITH INTEGRAL QUANTIFIED REAGENTS AND METHOD OF MODULATING SPECIMEN DRYING TIME**
BEWEISSAMMLER MIT INTEGRALEN QUANTIFIZIERTEN REAGENZIEN UND VERFAHREN ZUR MODULATION DER PROBENTROCKNUNGSZEIT
COLLECTEUR DE PREUVES POSSÉDANT DES RÉACTIFS QUANTIFIÉS INTÉGRÉS ET PROCÉDÉ DE MODULATION DU TEMPS DE SÉCHAGE DES ÉCHANTILLONS

(30) Priority: 25.02.2011 US 201113035577; 24.02.2011 US 201113034541; 27.02.2010 US 714477
(43) Date of publication of application: 02.01.2013
(60) Divisional application: 15182175.8
(73) Proprietor: The Bode Technology Group, Inc, Lorton, VA 22079 (US)
(72) Inventor: SANGHA, Jangbir S., Overland Park, Kansas 66209 (US)
(74) Representative: Teipel, Stephan
(86) International application number: PCT/US2011/026527
(87) International publication number: WO 2011/106784

(56) References cited:
- US-A- 4 014 748
- US-A- 4 175 008
- US-A- 4 211 323
- US-A- 4 223 093
- US-A- 4 813 432
- US-A- 4 873 193
- US-A- 4 917 867
- US-A- 5 856 172
- US-A1- 2002 057 991
- US-A1- 2003 113 906
- US-A1- 2004 082 878
- US-A1- 2005 009 200
- US-A1- 2007 255 175
- US-A1- 2007 255 175
- US-A1- 2007 299 364
- US-A1- 2007 299 364
- US-A1- 2008 206 740
- US-A1- 2008 254 550
- US-A1- 2009 043 226
- US-A1- 2009 215 159
- US-B1- 6 187 269

## Description

### Field of the Invention

The field of the invention is directed to apparatus and methods for field collection and transport and analysis of laboratory specimens and crime scene evidence samples. The field of the invention also relates to a method of modulating the drying time of such specimens or evidence samples after collection to achieve rapid drying of the specimens or evidence samples based on the quantity of specimen and the quantity of moisture present in the specimens or evidence samples.

### Background of the Invention

The present embodiments provide a specimen collection and drying and transport and storage device that can be used for laboratory and forensic purposes to gather samples and/or specimens and to then dry the sample and/or specimen during transport and/or storage prior to testing of the sample or specimen. All this can be accomplished in the present embodiments while providing assurance that the chain of custody has been preserved and that the collected specimen or sample has not been switched during the changing of the drying agent employed to dry the specimen.

More particularly, the embodiments relate to a specimen collection apparatus for collecting such samples and stabilizing the specimens and preserving them from contamination prior to laboratory analysis. Therefore, an apparatus is provided in which the specimen collector is enclosed after collection of the sample thereon to protect the sample from contamination. The embodiments also allow exposure of the specimen or evidence sample to a drying agent to dry and stabilize the specimen to promote specimen integrity by providing rapid drying soon after specimen collection. Further, the embodiments allow the user to renew, or change-out, exhausted drying agent without disturbing the specimen. And, the embodiments allow the user to select and insert variously sized desiccant packets to modulate the drying time of the collected specimen or sample depending upon user desires for the particular specimen or sample.

In one embodiment simultaneous, identical, dual specimen or sample collection is provided which allows two identical specimens to be simultaneously collected in one motion by the user and to then simultaneously deliver the dual and identical specimens to a single housing to thereby assure that the specimen or evidence samples receive simultaneous and identical protection, drying conditions and transport conditions. Further, the embodiment allows one of the two identical and simultaneously collected specimens to remain untouched or unused and to be archived without removal of the specimen from the original housing into which it was inserted after collection.
This may be accomplished while allowing the other of the two identical and simultaneously collected specimens to be removed from the housing or for a portion thereof removed for testing.

Crime scene evidence is collected to establish facts related to a crime or a suspected crime and for identification and/or elimination of suspects and may be presented at a trial for the determination of guilt or innocence of accused individuals. Often, the evidence includes objects, documents, fingerprints, photographs of the scene, and the like. Additionally, the evidence may include unknown substances or substances with a suspected identity, where the identity needs to be determined or confirmed. Such substances may be very small in quantity, may be dispersed over a comparatively large area, and may include materials such as: body fluids, hairs, flakes of skin such as skin cells, fibers, drugs, various chemicals, gunpowder residue, flammable materials, tobacco ashes, cosmetics, and the like. Such materials may be collected at a scene and subjected to chemical and/or DNA analysis for identification or for association with a particular individual.

Currently, for collecting specimen samples, investigators typically use fibrous swabs, such as swabs made of fibers of cotton, cellulose, rayon, polyester, polyester foam and other types of fibers. Such swabs not only absorb liquids and solids suspended in liquids but also trap dry substances such as particulate materials. Prior to use, the swabs are kept in closed sterile bags or containers to maintain sterility. After specimen collection the swabs and are placed into a similar bag or container to avoid contamination of the sample gathered during transportation. Once the swab is placed in a container after specimen collection, the container is usually marked with a time, the date, the identity of the investigator and other information to establish a chain of custody of the sample.

Conventional swabs are formed of a "stick" such as a shaft of wood, tubular plastic, or tubular or rolled paper with a pad of cotton or other fiber, sponge material, or other absorbent material attached to the end of the shaft, either mechanically or by an inert adhesive. A problem with conventional swabs is that there is a danger of contamination of the sample if it is necessary to put the swab down, for example, to open a bag or container in which the swab will be placed. Also, if it is necessary to set the swab down to dry, in a propped up condition or extending over the edge of a table, there is a risk of contamination of the sample.

The present embodiments provide an apparatus and method for collecting solid, fluid or particulate evidence specimens related to any type of situation in which evidence collection is required. Such evidence collection can be associated with crime scenes or can simply be the collection of a DNA sample from a human being in the course of a traffic stop or a paternity investigation. Suitable specimens for collection using the present devices are, in general, that evidence which is located on a surface or on a human being and which can be physically contacted by an evidence collection device to thereby obtain a sample of the evidence. Examples of such evidence specimens might be any type of biological fluid, either wet or dried, such as blood, urine or saliva, or any unknown substance which is visible or invisible and which can be located allowing for collection of a specimen of the evidence and capture of such a sample on a specimen collector of the type described hereinafter. As previously mentioned, it will be appreciated that such specimen collection devices are widely used in criminal investigations, but also are used increasingly in traffic stop situations or traffic arrest situations in which it is desirable to obtain a DNA sample from the suspect as part of a criminal records database requirement.

Therefore, for proper evidence collection that can be used in court to support a conviction, it is necessary that investigators have at their disposal a device and method of collection that dries the collected specimen shortly after collection to promote sample integrity by stabilizing the specimen by drying. It is additionally important that the apparatus promotes accuracy of specimen collection and reproducibility of specimen collection and protection of specimens from contamination while providing a device that enables a verifiable chain of custody while allowing continuous renewal of drying agents positioned adjacent to the specimen and while providing quantified specimen dilution during collection procedures and all without contributing to contamination of the crime scene by introducing extraneous material into the crime scene.

US 2007/255175 relates to a low-pressure sample collection apparatus including a double-ended cap member and a container member having one end closed and an opposite end open.

US 2007/299364 describes a containerized low pressure sample collection apparatus including an elongated tubular shaft with a vacuum connection at one end and a swab at an opposite end.

US 2003/113906 refers to a device for collection of DNA-containing materials.

### SUMMARY OF THE INVENTION

The present invention provides a specimen collection, drying, and transport apparatus comprising a specimen collector and a housing as defined in claim 1, and a method of specimen collection as defined in claim 10..

As described herein, the present device may further providing the crime scene investigator with interchangeable, quantified specimen collection reagents and variable specimen collection reagents, which due to the device structure are fully and accurately absorbable by the specimen collection swab.

According to the invention, the collection device provides for a swab on a specimen collector which swab can be conveniently detached from the specimen collector and specifically from the shaft connecting the swab to the specimen collector through use of a coaxially mounted tube which surrounds the shaft on which the swab is mounted. The coaxially mounted tube is provided with a terminal end which is located proximate to an area on the shaft where it is desired to have a point of breakage, or break-point location on the shaft, to separate the swab from the shaft to allow the swab to be separated from the specimen collector and to allow the swab to be deposited within a separate container. Another embodiment allow the swab to be pushed off the shaft by the use of the coaxially mounted tube. Yet another embodiment is provided with dual specimen collectors to allow simultaneous collection of identical specimens onto separate swabs. Furthermore, as described herein is a reagent vial cap retaining stand or projection to provide a specific, reproducible storage location for placement of the vial cap to avoid introduction of the cap into the crime scene by an investigator removing the cap from a reagent vile and placing the cap on a surface that in or adjacent to the specimen to be collected and part of the crime scene.

### Description of the Drawings

Fig. 1 is a top, front and right side perspective view of an embodiment showing the swab removed from the holder and the swab reversed and inserted into the neck of the holder to allow the holder to act as a handle for the swab during specimen collection procedures and showing fixed desiccant retainers holding the desiccant packets at a set distance from the area occupied by the swab when it is inserted into the holder;
Fig. 2 is a bottom, back and left side exploded view of the embodiment of Fig. 1 showing the desiccant chamber cap spaced from the desiccant chamber and two desiccant packets removed from the desiccant chamber and showing within the desiccant chamber the fixed desiccant retainers or guards that hold the desiccant packets at a specific distance from the swab while allowing insertion of desiccant packets into desiccant chamber and showing the swab aligned for insertion into the holder for drying, transport and protection from contamination;
Fig. 3 is bottom, back and left side perspective view of an embodiment showing the swab inserted into the holder where is becomes positioned between the desiccant packets held in the desiccant chamber to permit drying of a specimen collected on the swab during storage and transportation of the swab within swab holder to a laboratory for analysis of the specimen;

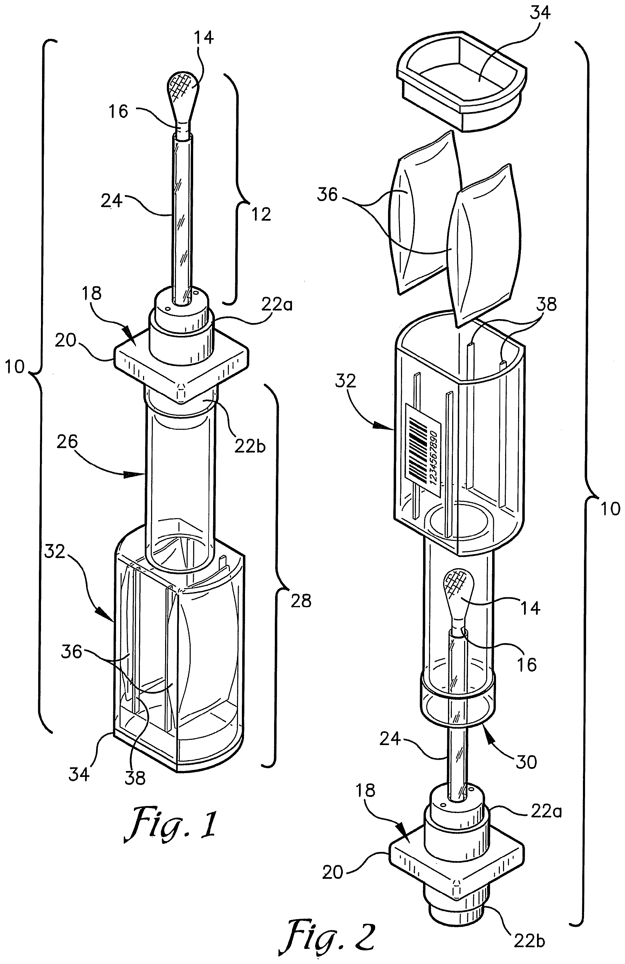

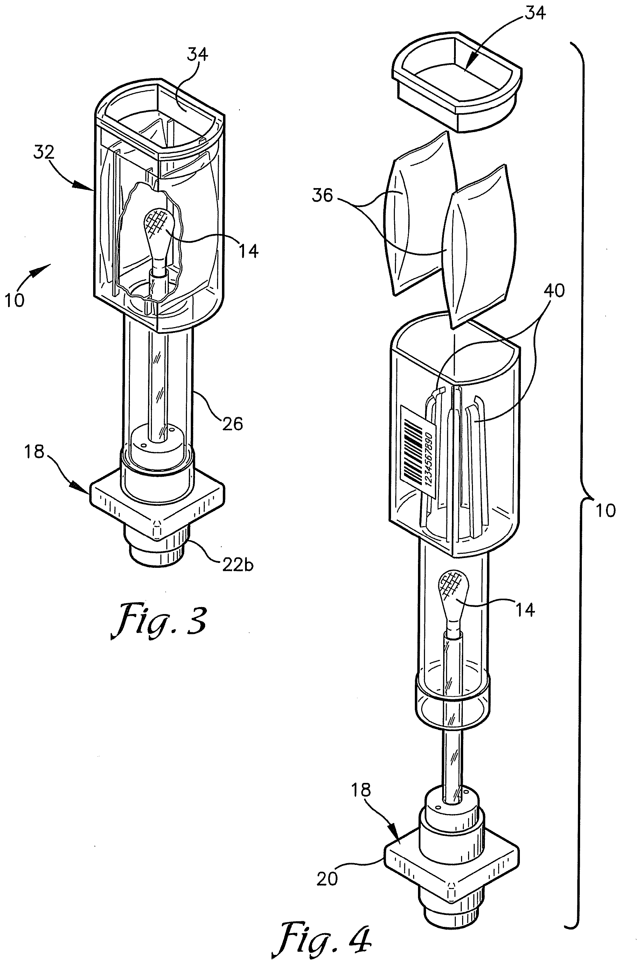

Fig. 4 is a bottom, back and left side exploded view of an embodiment similar to that of Fig. 2, but showing the desiccant being retained by multiple flexible retainers or guards that accommodate desiccant packets of various sizes and allow variation in the distance between the swab and the desiccant packet thereby allowing for variation in the speed of specimen drying and allow for accommodation of specimens of greater volume which may require a larger amount of desiccant in the packets to achieve the desired degree of specimen dryness during transport of the collected specimen in the holder;
Fig. 5 is a bottom plan view of the desiccant chamber of the holder showing
   the swab positioned within fixed retainers or guards, the retainers or guards being spaced from the swab and any specimen on the swab to keep the specimen out of contact with the desiccant packets during drying and/or transport and/or storage;

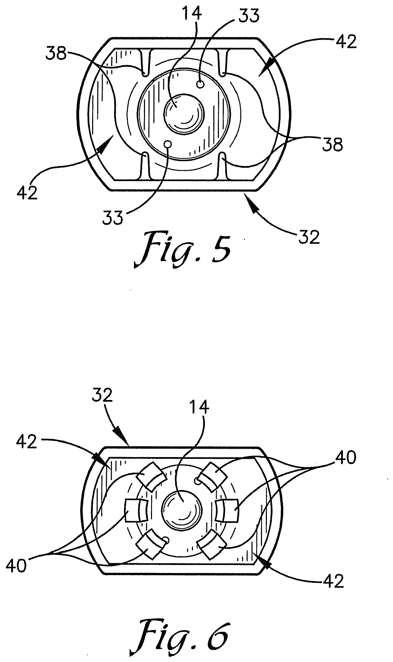

Fig. 6 is a bottom plan view of the desiccant chamber of the holder showing the swab positioned within a set of flexible retainers or guards the retainers being spaced from the swab, but being flexible at the point where the retainers contact the holder to permit the flexible retainers to accommodate variously sized desiccant packets to allow for variations in desired specimen drying time and variations in the specimen liquid content which can affect drying time as well as allowing for variation in the distance of the specimen from the desiccant which can change the drying time during storage and/or transport of the specimen.
Fig. 7 is a top, front and right side perspective view of an embodiment similar to that shown in Figs. 1-6 and having reagent holders mounted thereon;
Fig. 8 is a bottom rear and left side perspective view of a first variation of the device and showing a "T-shaped" securing structure on the bottom of the embodiment for holding a vial to the bottom of the embodiment;
Fig. 9 shows a bottom and front and left side prospective view of a second variation of the device and showing a friction-fit "C-shaped" securing structure for holding a vial to the bottom of the embodiment;
Fig. 10 is a cross-section view taken along line 10-10 of Fig. 9 and showing of the embodiment shown in Fig. 3 with the vial inside the exterior container held by the "C-shaped securing structure and showing the solid construction of central section or central member **20** which may be drilled through if desired to provide gas communication through the closure;
Fig. 11 is a cross-section view taken along line 11-11 of Fig. 7 and showing reagent vials within the reagent holders and also showing the solid construction of central section or central member **20** which may be drilled through to provide gas communication through the closure;
Fig. 12 is a left side, front and bottom perspective view of another embodiment showing a reagent vial held in the bottom of the embodiment;
Fig. 13 is a cross-section view taken along line 13-13 of Fig. 12 and showing the insertion of the reagent vial into a cavity in the bottom of the device and held there by a frictional fit;
Fig. 14 is a front right side and top perspective view of an embodiment of the embodiment having a vial formed in the sides of the device and a cap thereon with the structure of the embodiment walls also forming the walls of the vial;
Fig. 15 is a cross-section view taken along line 15-15 of Fig. 14 and showing the formation of the vials on the front and back sidewalls of the embodiment and showing the solid construction of central section or central member 20 which may be drilled through to provide gas communication through the closure;
Fig. 16 is a front, right side and top perspective view of an alternate embodiment of the embodiment of Fig. 1 and having a vial and cap insert that can be placed into a securing sleeve on the embodiment and having a cap receptacle for holding the vial cap to avoid contamination of a crime scene through the introduction of external materials into the crime scene such as the cap that closes the vial of the present embodiment;
Fig. 17 is a front, right side and top perspective view of the embodiment of Fig. 22 and showing the cap removed from the vial and placed on the cap receptacle to hold the vial cap to avoid contamination of a crime scene through the introduction of external materials into the crime scene such as the cap that closes the vial of the present embodiment;
Fig. 18 is a front, right side and top perspective view of an alternate. embodiment and showing the cap receptacle for holding the vial cap included as part of the cap that seals the body of the container;
Fig. 19 is a front, right side and top perspective view of the embodiment of Fig. 18 and showing the cap removed from the vial and placed on the cap receptacle that is positioned on the cap that seals the body of the container;
Fig. 20 is a front and top perspective view of the vial and cap insert that may be used with the embodiment of Figs. 24 and 25 and other embodiments;
Fig. 21 shows an embodiment having dual swabs on dual shafts with each shaft having a break-off tube coaxially mounted on the shaft to allow for simultaneous, dual specimen collection by a user and showing the alignment indicator and closure rotation lock on the closure and on the holder that allows the user to properly align the dual swab collector on the holder to provide proper spacing of the swabs from the desiccant and showing dual vial carriers made integrally with the body of the device and showing a closure rotation indicator and locking structure on the neck of the embodiment;
Fig. 22 shows a cross-section view of the embodiment of Fig. 21 taken along line 22-22 of Fig. 21 and showing the neck of the embodiment of Fig. 22 having the closure rotation indicator and locking structure **74** on the neck of the embodiment engaged with the closure rotation lock **75** on closure **18;**
Fig. 23 is a front and top perspective view of a vial and cap insert that may be used with the embodiment shown in Figs. 27 and 28 and other embodiments;
Fig. 24 shows an exploded perspective view of an embodiment of a closure which can be used with the embodiments described herein and having a reagent vial insertable into the closure for transport of a swab solution therein;
Fig. 25 shows a swab being separated from the shaft by use of a break-off tube coaxially mounted on the swab shaft.

### Detailed D scription

As required, detailed embodiments of the present inventions are disclosed herein; however, it is to be understood that the disclosed embodiments are merely exemplary of the invention, which may be embodied in various forms..

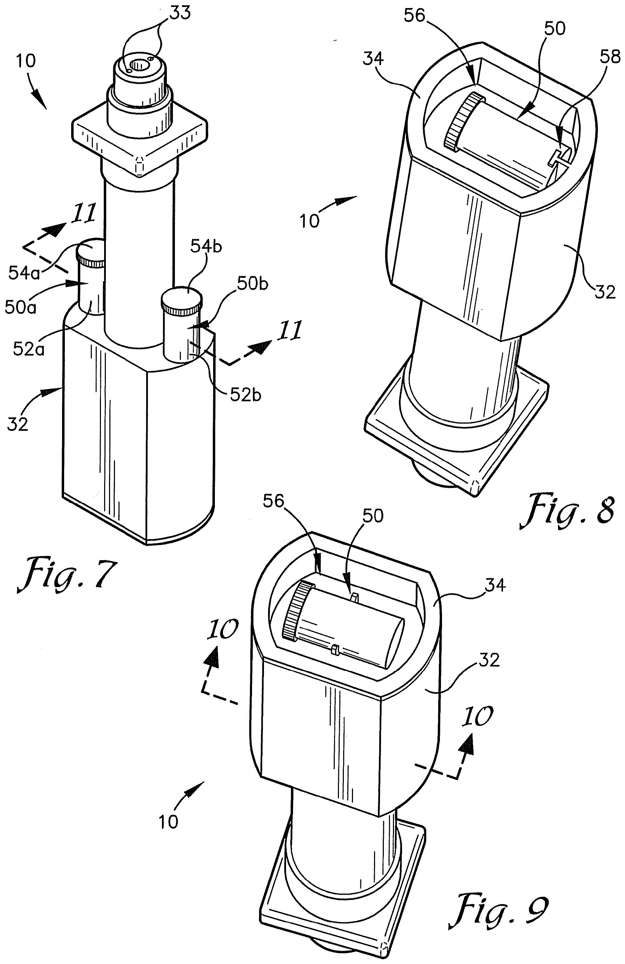

Figures 1 - 24 relate to embodiments of a unitized apparatus for collection and/or drying and/or transport and/or analysis apparatus **10** and a method for modulating drying time of the specimen through the use of user selectable and user sizeable desiccants and user renewable desiccants. Apparatus **10** comprises, generally, a swab mounted on a shaft, the shaft connected to a closure, and a housing or holder having a drying chamber containing a desiccant. The embodiments shown in Figs. 1-6 are generally similar in construction but different in the means by which the desiccant is retained within the holder. The embodiments of Figs. 7-19 include quantified reagent holders.

First referring to Figs. 1-4 the unitized apparatus for collection and/or drying and/or transport and/or analysis apparatus **10** will be described. In Fig. 1 specimen collector **12** comprises a swab **14** mounted on a first end of a shaft **16** with the second end of the shaft connected to a closure **18.** The closure **18** comprises a central member
**20** having a stopper **22a, 22b** extending from each of the two opposed sides of the central member. The specimen collector **12** further comprises a break-off tube **24** mounted coaxially on the shaft **16.** The tube **24** is formed of a material that has greater rigidity than the material used to form shaft **16.** A first end, of tube **24** is connected to closure **18** and a second end of tube **24** is configured to terminate at a selected location along shaft **16** at which it is desired to break shaft **16** to achieve separation of swab **14** and the portion of the shaft to which swab **14** is mounted from the remainder of shaft **16.** This location on shaft **16** is referred to as the break-point location and will vary depending on the length of break-tube **24** that is mounted on shaft 16. Alternatively the break-tube **24** may be connected into closure **18** in a separable manner to allow tube **24** to be pulled from connection with closure **18** and pressed along or slid along shaft **16** until it contacts swab **14** whereupon it can be used to force swab **14** off of shaft **16** and into a container or other receptacle.

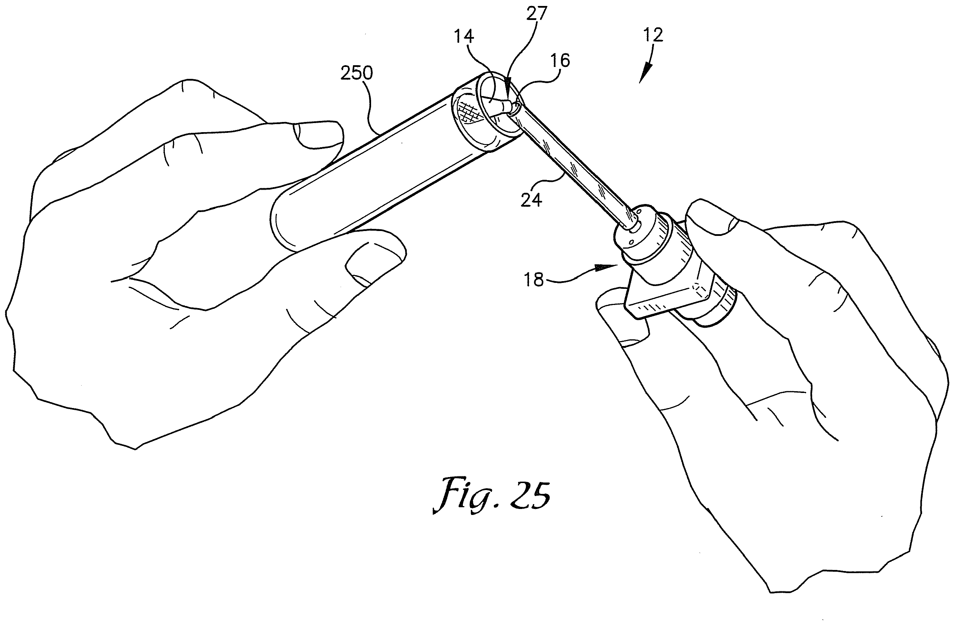

For clarity this type of separation of swab **14** from specimen collector **12** is shown in Fig. 25. In Fig 25, it may be seen that swab **14** is pressed against a solid surface such as the side of container **250** and a bending motion is applied by the user to press swab **14** back toward tube **24** and closure **18.** Upon sufficient pressure being applied, the shaft **16** will break at or near the terminus of tube **24.** Then swab **14** and the portion of shaft **16** to which swab **14** is connected will separate from the portion of shaft **16** that is connected to closure **18.** This allows the swab and the specimen that is collected onto the swab to be separated from the remainder of device **10** and separately placed into a reaction tube for analysis and/or an alternate container for shipment.

Again referring to Fig. 1 the closure **18** comprising central member **20** and having a stopper **22a, 22b** extending from each of the two opposed sides of the central member is shown with stopper **22a** having shaft **16** and tube **24** connected thereto and with stopper **22b** inserted into neck **26** of housing **28** of
apparatus **10.** Fig. 1 presents the embodiment in its open position. In the open position, specimen collector **12** has been removed from housing **28** and the closure **18** has been reversed and inserted into opening **30** (Fig. 2) of the neck **26** of housing **28** from which closure **18** and swab **14** on shaft **16** and break-off tube **24** were just removed. This reversal and insertion allows housing **28** to act as a handle for manipulating the swab **14** of specimen collector **12** during the collection of a specimen onto swab **14.** The relatively large, flat surface of desiccant chamber **32** fits securely into the palm of the hand and provides a flat surface that will prevent rolling of the apparatus **10** if it is placed on a surface. When positioned on a surface the edge of closure **18** extends laterally beyond swab **14** and keeps swab **14** separated from any contact with adjacent contaminating surfaces. The closed position for apparatus **10** is shown in Fig. 3 wherein specimen collector **12** has been inserted into housing **28** and stopper **22a** of closure **18** has been inserted into opening **30** (Fig. 2) of the neck **26** of housing **28** so that stopper **22a** of closure **18** having swab **14** on shaft **16** and break-off tube **24** connected thereto all are inserted into housing **28.**

Referring now to Figs. 2 and 4 the housing **28** being further comprised of desiccant chamber **32** connected to neck **26** of housing **28,** will be described. Desiccant chamber **32** is provided with resealable cover **34** that forms the bottom of housing **28.** Cover **34** may be generally flat to allow apparatus **10** to stand on a surface. Cover **34** may be removably connected to desiccant chamber **32** or it may be permanently sealed to close chamber **32.** It will be appreciated that the permanent sealing of chamber **32** by cover **34** may be accomplished at the time of manufacture or upon the insertion of a specimen on swab **14** into housing **28** or cover **34** may be used to permanently seal housing **28** at any time thereafter. Desiccant packets **36** of Figs. 2 and 4 have been removed from desiccant chamber **32** to better show the fixed retainers **38** (Fig. 2) and flexible retainers **40** (Fig. 4) that hold desiccant packets 36 in position within desiccant chamber 32. It will be appreciated from the Figs. 2 and 4 that fixed retainers extend from a sidewall of desiccant chamber **32** and flexible retainers are a plurality of flexible finger-like structures that extend downwardly from the top of desiccant chamber **32** and can accommodate desiccant packets of various sizes and shapes by flexing toward and away from the swab isolation area **41.** When swab **14** is positioned within housing **28,** as shown in Fig. 3, it may be seen that swab **14** situated between the retainers **38** (Fig. 2) or within retainers **40** (Fig. 4) in a swab isolation area **41** with the retainers **38, 40** holding desiccant packets **20** away from swab **14.** The swab **14** is positioned between, but not contacted by, desiccant
packets **36** to avoid contamination of swab **14.**

In Fig. 4 an embodiment similar to that of Fig. 2 is shown in an exploded view. In Fig. 4 desiccant chamber cover **34** is separated from the desiccant chamber **32** and the two desiccant packets **36** have been removed from the desiccant chamber **32.** Visible within the desiccant chamber **32** are the flexible retainers **40** that allow variable spacing of the desiccant packets **36** from the swab **14.** It will be appreciated that the flexible nature of flexible retainers **40** allows insertion of variously sized desiccant packets **36** into desiccant chamber **32.** This is accomplished by the flexible retainers **40** being able to bend inwardly toward swab **14** to expand the distance between flexible retainers **40** and the walls comprising desiccant housing **32.** Due to this repositionable nature of flexible retainers **40,** user selectable quantities of desiccant and variable volumes of desiccant and variable sizes of desiccant packets can be introduced by the user into desiccant chamber **32** to change the drying time of a specimen captured on swab **14.** Desiccant packets **36** are positioned to be in close proximity to swab **14** to absorb moisture from the specimen that is collected on swab **14.** As the proximity of desiccant to moisture has a direct correlation to the rapidity of drying, it will be appreciated that the close, but spaced, proximity of the desiccant to swab **14** is particularly efficacious in speeding the drying of moisture that may be on swab **14.** Such variation is made possible by flexible retainers **40.** It also will be appreciated that resealable cover **34** permits the replacement of desiccant packets 36 at any time during the use of device **10** and without the need to disturb swab **14** and/or any specimen thereon.

In Fig. 3 a perspective view is shown of the device **10** of Figs. 2 and 4 with swab **14** inserted into housing **28.** In this position swab **14** is positioned between desiccant packets **34** for drying and is protected within housing **28** for transport and/or storage. It may be observed that swab **14** is positioned between guards **38.** In Fig. 3 a portion of the desiccant packets **36** have been removed and a portion of the wall of desiccant chamber **32** has been removed for clarity.

It will be understood that in Fig. 3, closure **18** has been reinserted into neck
**26** to dispose swab **14** and shaft **16** and break-off tube **24** within housing **28.** This positioning places swab **14** disposed between retainers **38, 40** and within desiccant chamber **32.** It will be appreciated that flexible retainers **40** extend beyond the bottom of swab **14** to prevent objects inserted into desiccant chamber **32** from making inadvertent contact with swab **14.** Those skilled in the art will appreciate that with desiccant chamber cover **34** removed, as shown in Fig. 4, that desiccant chamber **32** is open and accessible. It is in this configuration that desiccant packets **36** can be inserted, removed, renewed or increased or decreased in size by the user as may be indicated by the needs of the particular specimen on swab **14** or the need to speed up or slow down drying of the specimen on swab **14.** It also may be observed in Figs. 5 and 7 that closure **18** may be provided with air holes **33** that extend through closure **18.** Air holes **33** can aid in the drying of the specimen and air holes **33** can be excluded from the embodiment completely if desired.

In Fig. 5 the fixed or rigid retainers **38** and the swab **14** are shown from a bottom view into desiccant chamber **32.** In this view it may be seen that swab **14** is positioned between retainers **38** and spaced therefrom so as not to contact retainers **38** or the walls of desiccant chamber **32.** Desiccant holding areas **42** extending between retainers **38** and the walls of desiccant chamber **32** are best observed in Figs. 5 and 6.
It will be appreciated that variously sized desiccant packets **36** can be inserted into desiccant holding areas **42** during drying and/or transport and/or storage. Once the desiccant packets **36** have become exhausted by absorption of moisture they may be replaced. This is accomplished by removing cover **34** withdrawing exhausted desiccant packets **36** and inserting new desiccant packets **36.** Once replacement has been accomplished, the desiccant chamber resealable cover **34** may be replaced to again close desiccant chamber **32** to the outside.

In Fig. 6 the flexible guards **40** and the swab **14** are shown from a bottom view into desiccant chamber **32.** In this view it may be seen that swab **14** is positioned within flexible guards **40** and spaced therefrom so as not to contact flexible guards **40** or the walls of desiccant chamber **32.** It will be appreciated that the ends of flexible guard **40** bend inwardly to operate to deflect material, such as desiccant packets **36** when they enter desiccant chamber **32,** from contacting swab **14** and any specimen thereon. Desiccant holding areas **42** extending between flexible guards **40** and the walls of desiccant chamber **32.** It will be appreciated that as flexible guards **40** may be pushed away from desiccant chamber 32 walls that variously sized desiccant packets **36** can be inserted into desiccant holding areas **42** during drying and/or transport and/or storage. Once the desiccant packets **36** have been inserted, the desiccant chamber resealable cover **34** may be replaced to again close desiccant chamber **32** to the outside. It will be appreciated that the flexible guards **40** in particular allow the user to select and insert variously sized desiccant packets to modulate the drying time of the collected specimen or sample depending upon user desires for the particular specimen or sample. In addition the flexible guards **40** permit larger desiccant packet volumes to approach more closely to the swab 14 as it resides in the swab isolation area 41 since the flexible guards 40 can move inwardly toward the swab thereby placing the desiccant closer to the specimen. This configuration will modulate the drying of the specimen as the closer proximity of the desiccant to the moisture of the specimen on the swab will decrease the drying time of the specimen and enhance the stability of the collected specimen by drying the specimen faster.

Referring now to Fig. 7 an embodiment of a type shown in Figs. 1-6 is shown further comprising the addition of reagent holders mounted on the top of desiccant chamber **32.** Reagent holders **50a, 50b** extend from desiccant chamber 32 and are molded in unitary fashion with desiccant chamber 32. The reagent holders **50a, 50b** are comprised of a body **52a, 52b** and a cap **54a, 54b.** Caps **54a, 54b** may be of the screw type or the friction fit type of cap.

Referring now to Fig. 8 and Fig. 9, embodiments are shown having the reagent holders **50** mounted on desiccant chamber removable cover **34.** In the embodiment of Fig. 8, reagent holder **50** is held within an indention formed in cover **34.**
The indention being sufficient to allow the entirety of reagent holder **50** to sit within the indention while yet allowing apparatus **10** to stand on a flat surface with resalable cover **34.** Such contact with the surface is shown in Fig. 7. In Fig. 8, reagent holder **50** is retained within indention **56** by a tongue and groove shaped arrangement with the groove being within the bottom of the reagent holder **50** and the tongue extending from removable cover **34** and being configured to be mateable with the groove in the bottom of the reagent holder **50.** In Fig. 9, the reagent holder **50** is retained within indention **56** by C-shaped which provides a frictional fit capture of the reagent holder **50** within the C-shaped retaining clip.

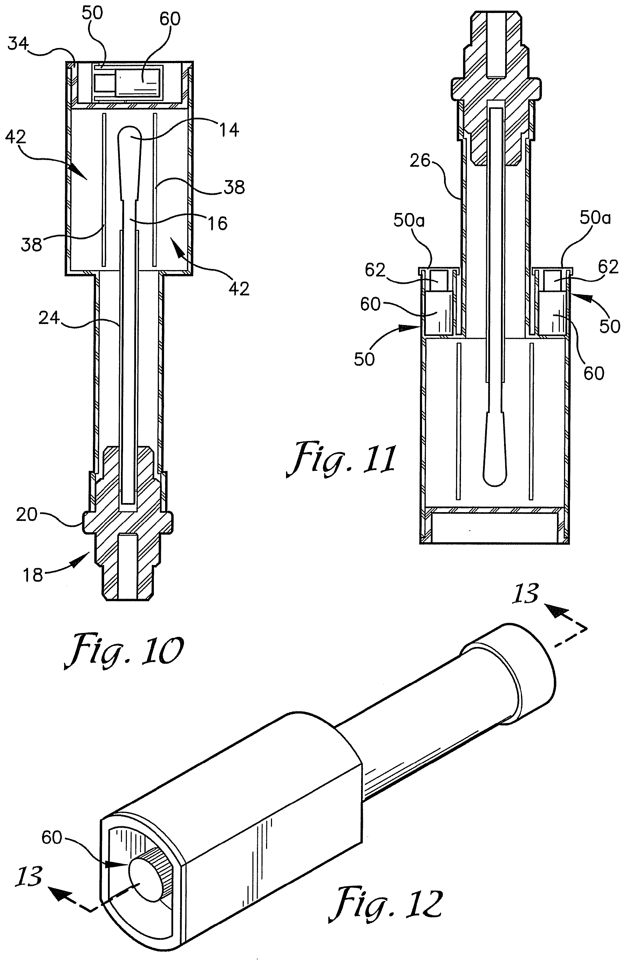

Referring now to Fig. 10, a cross-section view taken along line 10-10 of Fig. 9 is shown. In Fig. 10, it can be seen that a device of similar construction to the device shown in Figs. 1 and 2 is shown having desiccant holding areas **42** and retainers **38** and a swab **14** on shaft **16** having tube **24** coaxially mounted thereon. Also shown in Fig. 10 is reagent vial60 which is in reagent holder **50.** It will be appreciated by those skilled in the art that using a separate reagent vial **60** held within a reagent holder **50** that different reagent compositions and of different volumes may be rapidly and easily substituted into reagent holder **50** by simple substitution of a different reagent vial **60.**

Referring now to Fig. 11, a cross-section view taken along line 11-11 of Fig. 7 is shown. In Fig. 11, reagent holders **50** are shown to either side of neck **26** with each vial **60** having a cap **62** thereon and reagent holder **50** having its own cap **50a** serving to retain vial **60** within reagent holder **50.**

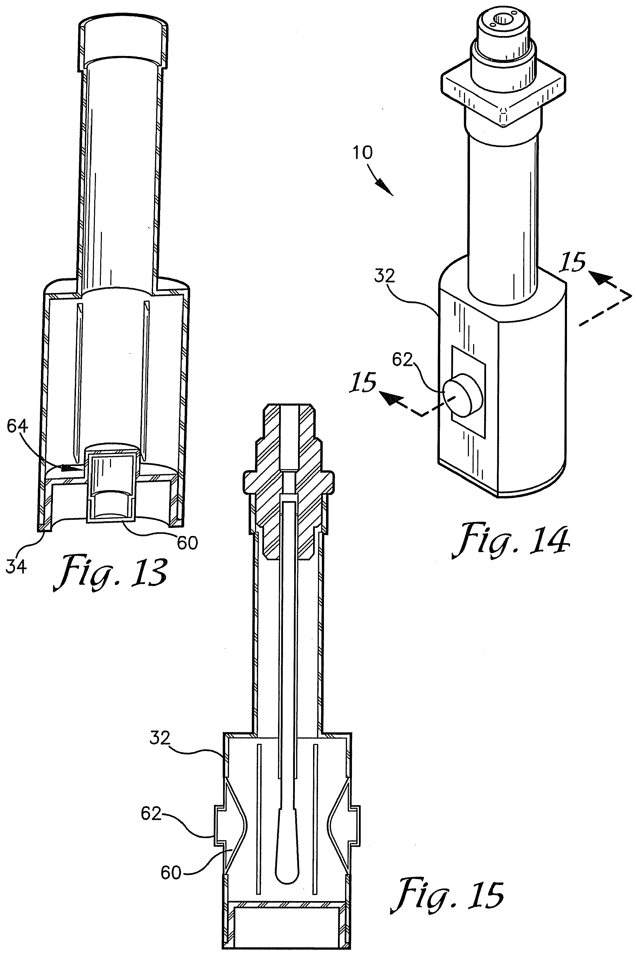

Referring now to Fig. 12, an embodiment is shown having reagent vial **60** inserted into a depression formed in the surface of desiccant chamber receivable cover **34.** In Fig. 13, a cross-section view taken along line 13-13 of Fig. 12 is shown. In Fig. 13, the cross-section view of the embodiment of Fig. 12 shows that cover 34 is provided with an indention **64** which is configured to capture vial **60** therein by a frictional fit between the bottom of vial **60** and the walls of indention **64.**

In Figs. 14 and 15, yet another embodiment of the reagent holder on the apparatus is shown. In Fig. 14, it can be seen that the reagent vial60 is formed integrally with the sidewall of desiccant chamber **32.** This may be more clearly seen in Fig. 15, which is a cross-section view taken along line 15-15 of Fig. 14. In Fig. 15, reagent vial **60** is shown as comprising an indention in the sidewall of desiccant chamber 32 and having cap **62** thereon to seal reagent vial **60.**

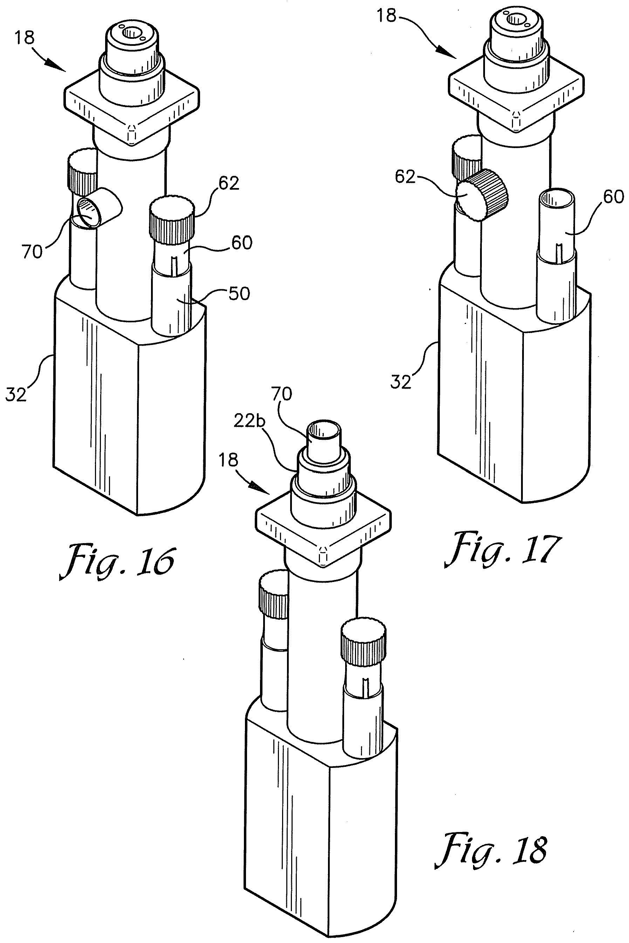

Referring now to embodiments shown in Figs. 16-20, embodiments having reagent holders and reagent vials are shown but also having the added advantage of having a cap stand included in the embodiment to retain a reagent holder cap or a reagent vial cap and to provide secure, reproducible placement in the keeping of the reagent or vial cap thereby to avoid loss of the vial cap and to avoid contamination of a crime scene in particular. The cap receptacle allows the evidence collection technician to avoid contamination of a crime scene by the inadvertent introduction of external materials into the crime scene. Specifically, the receptacle allows the cap that closes the vial to be placed in a specific, anticipated, repeatable location that is a part of the equipment brought to the scene by the evidence collection technician. In this manner the evidence collection technician will always know where to put the cap and where to locate it at the conclusion of the specimen collection. This provides a consistent and repeatable activity that can become a part of the evidence collection technicians method of practice and thereby reduce the introduction of external materials and potential extraneous DNA that might contaminate the crime scene.

Referring now to Fig. 16 and 17, an embodiment is shown having a cap stand **70** extending from neck **26 of** holder **28.** In Fig. 17, it can be seen that a cap **62** has been removed from reagent vial **60** and has been placed onto cap holder **70** where cap **62** is retained during the course of a collection procedure performed with the embodiment shown in Fig. 17. It also will be appreciated that having the reagent holder **50** and reagent vial **60** positioned in upright fashion on the top of desiccant chamber 32 allows the investigator, particularly a crime scene investigator, to have the reagent contained in reagent vial60 available for use in wetting the swab **14** which is attached to closure **18** without a need to attempt to manipulate additional devices and structures to wet the swab **14** or to find a suitable location to place holder **28** within the crime scene to free a hand to hold the reagent vial **60** while wetting swab **14** of a specimen collector **12** with a suitable reagent such as that which is contained in reagent vial **60** for a specimen collection.

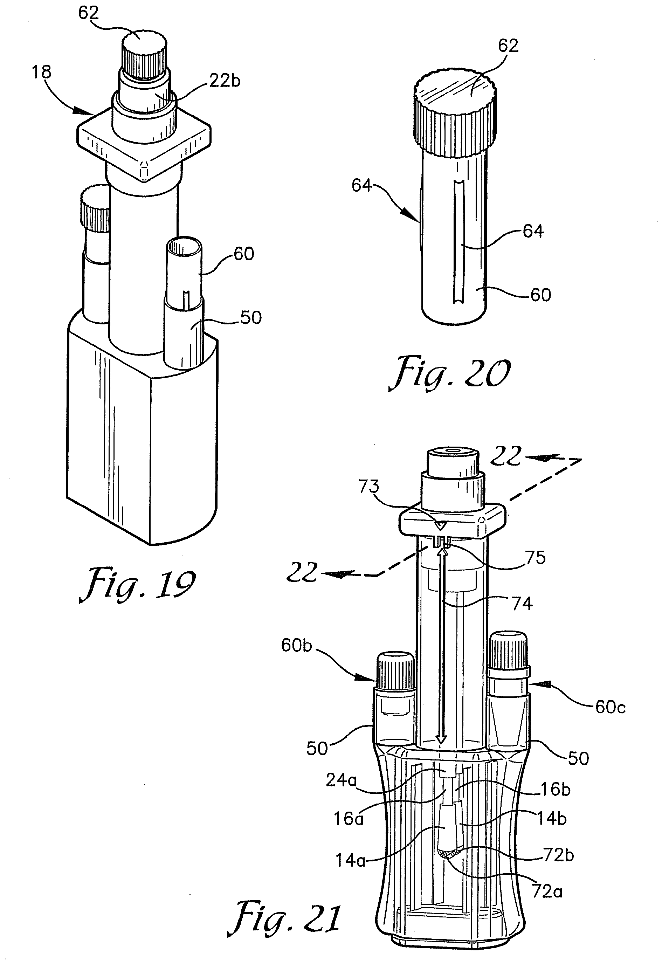

Referring now to Fig. 18, an alternate embodiment is shown and which is similar to the embodiments of Figs. 16 and 17 but in which the cap stand **70** is formed in the top of stopper **22b** of closure 18. It will be appreciated that the embodiment of Fig. 18 operates in similar manner to the embodiment described in Fig. 16 and 17. Such similar operation is shown in Fig. 19 wherein a cap **62** has been removed from a vial **60** and the cap **62** has been placed upon cap stand **70** which extends from stopper **22b** closure **18.** In Fig. 20, reagent vial60 is shown of the type used in many of the embodiments described herein. Vial **60** is provided with longitudinal projections **64** which are compressible and which enhance the friction fit of reagent vial **60** within reagent holder **50** and which allow the passage of air in and about the sidewall of reagent vial **60** and the sidewall of reagent holder **50** when the two are insertably joined together as shown in Fig. 19. The importance of this feature will be appreciated by those skilled in the art who have contended with a moisture seal between two closely fitted surfaces and the barrier to separation of the two structures caused by the moisture seal preventing the intrusion of air and causing a need to overcome a vacuum which is created between the two surfaces when the withdrawal of the objects from insertion, one within the other, is attempted. Projection **64** assists in such separation while also providing secure frictional fit between vial **60** and reagent holder **50.**

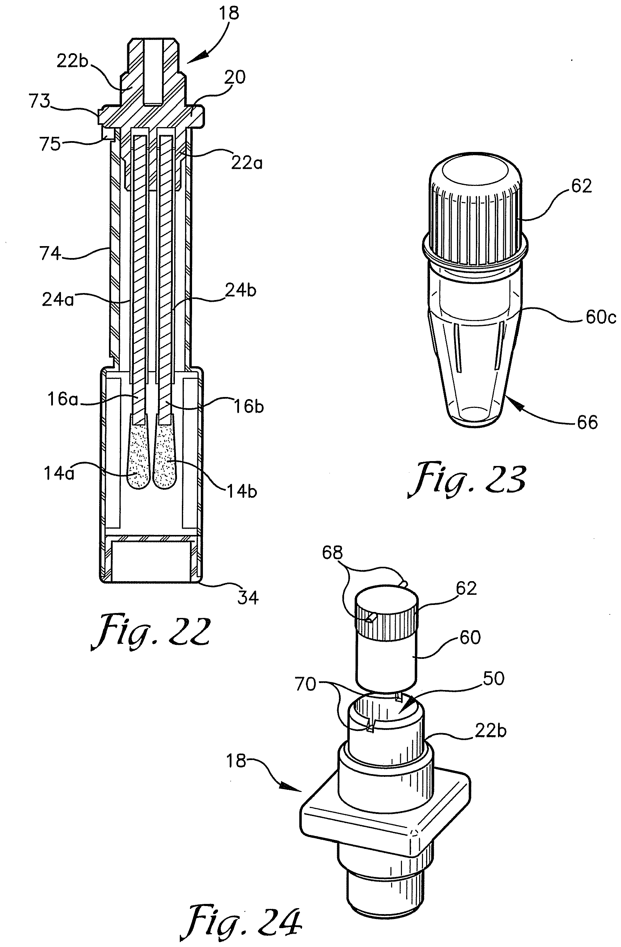

In Figs. 21 and 22 an embodiment is shown having dual swabs **14a,b** mounted on dual shafts **16a,b** and having dual break-off tubes **24a,b** coaxially mounted on each of the shafts. Both of these dual swab, shaft and break-off tube combinations are connected to the same stopper **22a** extending from central member **20** of closure **18.** The embodiment of Figs. 21 and 22 allows the user to collect simultaneously, identical, dual specimens or samples **72a,b** in one motion or in a single contact with a specimen or evidence location. Then the user can simultaneously deliver the dual and identical specimens **72a,b** to a single housing **28** to thereby assure that the specimen or evidence samples receive simultaneous and identical protection and drying conditions and transport conditions are provided to the identical, dual specimens. The embodiment of Figs 21 and 22 permits a user to remove one of the two identical and simultaneously collected specimens **72a,b** while allowing the other specimen or sample **72a,b** to remain untouched or unused and to be archived without removal of the specimen from the original housing into which it was inserted after collection. This simultaneous, dual collection and protection of a specimen or evidence sample is of great importance for evidence collection as it allows collection of two identical specimens **72a,b** under exactly the same conditions, from exactly the same location of the evidence, and permits the separate removal and testing of one of the dual identical specimens without any change or disturbance to the other specimen and while leaving one of the dual identical specimen fully intact and untouched for archiving and further or future testing. This can be highly important in providing a second identical specimen for test verification where an analysis method that is destructive of the specimen must be employed.

Fig. 21 an embodiment is shown having dual swabs **14a,b** connected to dual shafts **16a,b** and with each shaft having a break-off tube **24a,b** coaxially mounted on the shaft. As previously described for Fig. 25, the break-off tubes **24a,b** allow for the swab **14** to be separated from the shaft **16.** It also will be appreciated that the embodiment of Figs. 21, 22 is provided with desiccant packets 36 in desiccant chamber **32** to permit simultaneous, and identical drying conditions for the dual specimens. As previously described, when swabs **14a,b** are positioned within housing **28,** the swabs **14a,b** are to be situated between the retainers **38** (Fig. 2) or within retainers **40** (Fig. 4) with the retainers **38, 40** holding desiccant packets **20** away from swab **14.** It will be appreciated that it is important that swabs **14a,b** be positioned between, but not contacted by, desiccant packets **36** to avoid contamination of swabs **14a,b.** To assure the proper location of swabs **14a,b** the embodiment of Figs. 21, 22 is provided with alignment indicators on closure **18** and holder **28.** In Figs. 21 and 22 closure **18** is provided with indicator 73 on central member **20** and holder **28** is provided with indicator **74.** In operation, a user upon inserting specimen collector **12** into holder **28** will observe the alignment of indicators 73 and **74** and then rotate closure **18** within holder **28** until the indicators **73, 74** are aligned one above the other as shown in Fig. 21. This alignment assures that the swabs **14a,b** are positioned between retainers **38 or 40** in a
position that provides uniform separation between each of swabs **14a,b** and desiccant packets **20.** In this manner the identical drying of swabs **14a,b** is assured.

The embodiment of Figs. 21 and 22 also includes a closure rotation lock 75 on the closure **18.** During insertion of specimen collector **12** into holder **28** and after alignment of indicators **73, 74** the closure can be pressed downwardly into holder **28** to insert holder indicator **74** into closure rotation lock 75 to thereby prevent inadvertent rotation of specimen collector **12** within holder **28.** In this manner the proper alignment of the dual swab collector on the holder to provide proper spacing of the swabs from the desiccant is assured during future use and transportation.

It will be appreciated that the embodiment of Fig. 21 and 22 can be used to capture evidence at a crime scene that may be used as a control during analysis while providing exactitude in the identical handling of the control swab since both the control swab and the specimen swab are handled simultaneously during the collection and drying and transport phases of evidence collection and the evidence security will be identical for both specimens. In the case that one of the dual swabs may be a control the evidence collector would use a first of the two dual swabs to take a specimen of the area surrounding the evidence specimen of interest. Then the second swab would be used to obtain a sample of the evidence specimen as it existed in the crime scene. Then both swabs would be treated identically and simultaneously during the remainder of the collection and insertion into the housing and marking and evidence security and shipping procedures. If a specimen containing DNA was collected on the evidence swab, the control swab could be examined to determine if background DNA was present in the vicinity of the DNA evidence and if background DNA was present on the control swab the background DNA then could be removed from the analysis of the DNA found on the evidence swab.

A further use of the dual swab embodiment may be to provide simultaneous, wet/dry specimen collection from a single evidence specimen after which both swabs may be treated identically and simultaneously during the remainder of the collection and insertion into the housing and marking and evidence security and shipping procedures. In this method of collection one of the dual swabs is wetted with a reagent contained in one of the reagent vials **60** which contains a wetting reagent therein. The wetted swab is then applied to the evidence of interest and used to both collect a wetted sample from the evidence. This procedure will result in a wetted evidence of interest after which the other swab of the dual swabs, the dry swab, may be applied to the now wetted evidence of interest to further collect a sample of the evidence of interest.

Also shown in Fig. 21, the provision for both a reagent holder **50** and a separate reagent vial **60** will be appreciated for allowing the use of variously sized reagent vials **60** which can contain precisely measured but different volumes of reagent to be applied to either swab **14** or to a specimen to be collected. As shown in Fig. 21, vial **60b** is substantially smaller than is vial **60c.** In providing individual vials for the provision of reagents to be applied to swab **14,** the benefit is provided that exact quantization of the dilution of a specimen that is collected can be determined. In the prior art typical swab wetting procedure, an absorbent swab is held beneath a container nozzle and the technician attempts to apply individual drops of a reagent to the swab. The usual result is that the first drop or drops or substantial portions thereof bead up and fall off the swab due to the swab surface not being immediately absorbent As described therein, by providing an actual vial holding a reagent, the swab can be dipped into the vial where the pre-measured optimum quantity of a user selected
reagent is held in contact with the swab **14** and complete absorption of the reagent onto the swab is accomplished. This absorption is further assisted by the pressure that can be brought to bear on the swab by the sidewalls of the vial 60 pressing against the swab **14** to assist in overcoming the surface tension present on the swab **14** thereby assisting in overall absorption of the reagent contained in vial60. In Fig. 23, a vial of the type shown inserted in the reagent holder **50** of Fig. 21 is shown in greater detain and having inverted conical sidewalls 66 which further assists in the complete absorption of a small volume of reagent liquid on to swab 14. It will be appreciated that depending on what specimen is to be collected or what specimen is of interest to the investigator that the quantity and type of reagent in the vial may be user selected. For example if it is of particular interest the semen be immediately identified if it is present in the crime scene then the user or evidence technician can insert vials into the reagent holders that contain a semen reactive reagent to identify the presence of semen upon the swab contacting semen in the crime scene evidence. Or, if blood is of particular interest the evidence collection technician can insert vials into the reagent holders that contain a blood reactive reagent to identify the presence of blood upon the swab contacting the unknown crime scene specimen.

The quantified reagent vials **60** which are interchangeable within the reagent holders **50** are configured to provide a reproducible, quantitative wetting of the swab with a known amount of solution and which results in the wetting of the swab by a known volume this provides a quantified absorption of reagent onto the swab which is not possible with previous devices. As described above, the past procedures of attempting to add reagent in a drop-wise manner onto the swab could not produce a swab having a known quantity of reagent on the swab due to loss of drops or loss of portions of drops from the swab surface prior to absorption of the drop by the swab.

Fig. 24 shows a closure **18** having a reagent holder **50** formed into a stopper 22b for insertion of a vial **60** therein and with cap **62** of vial **60** being provided with flanges **68** which are captured within detents **70** of stopper **22b** which assists in drawing vial **60** from stopper **22b** as cap **62** will, when inserted into stopper **22b,** be flush with the top of stopper **22b.**

In Fig 25 the method by which swab **14** is separated from shaft **16** by applying the terminal end of break-off tube **24** to a break-point **27** located on shaft **16.** In Fig. 25 swab **14** is pressed against the side of container **250** and a bending motion is applied by the user to press swab **14** back toward tube **24** and closure **18.** When sufficient pressure is applied shaft **16** will break at or near a break-point **27** which is adjacent the terminus of tube **24** as it is the terminus of tube **24** which establishes to point of application of bending force to shaft **14.** When sufficient force is applied, shaft **16** will break and swab **14,** and the portion of shaft **16** to which swab **14** is connected, will separate from the portion of shaft **16** that is connected to closure **18.** This allows the swab and the specimen that is collected onto the swab to be separated from the remainder of device **10** for analysis and shipment. Alternatively, the break-off tube may be used as a swab pushed-off device. In this instance the break-off tube may be pushed by the user along the shaft to slide the break-off tube into contact with the swab. The break-off tube in this embodiment of configured to be a close, but slideable coaxial fit on the shaft and sufficiently smaller in diameter than the swab that the break-off tube will not slide over the exterior of the swab. In this embodiment the break-off tube will contact the swab and be used by the user to press the swab off the end of the shaft and into a reaction container or other tube or holder or shipping container.

## Claims

1. A specimen collection, drying, and transport apparatus (10) comprising:
a. a specimen collector (12) comprising:
a specimen collection swab (14) said swab connected to a shaft (16) having a first shaft end with said specimen collection swab (14) thereon and a second shaft end connected to a closure (18), said closure having first (22a) and second (22b) stopper structures extending from opposed sides of a central member (20), said first stopper structure (22a) connected to the shaft (16); and
a break-off tube (24) mounted coaxially on the shaft (16), the break-off tube having a first end connected to said first stopper structure (22a) and a second end terminating at a shaft break-point (27) on said shaft (16), said shaft-break-point (27) being positioned along said shaft (16) at a location sufficiently spaced from said swab (14) to permit the entire swab (14) to be separated from the shaft (16) when the shaft break-point (27) is pressed against the break-off tube (24);
b. a housing comprising:
a desiccant chamber (32);
a neck (26) extending from the desiccant chamber (32) the neck having an opening providing communication through the neck and into the desiccant chamber (32) for passage of the swab from the neck and into the desiccant chamber;
a swab isolation area (41) in the desiccant chamber (32) the swab isolation area (41) aligned with the neck to receive the shaft mounted swab from the neck and to position the swab in the swab isolation area (41) of the desiccant chamber (32);
a desiccant holding area (42) on at least two sides of the swab isolation area (41) the desiccant holding area (42) configured to retain desiccant packets (36) therein to absorb moisture from a specimen collected on the swab (14); and
an openable and closeable bottom (34) on the desiccant chamber configured to permit removal of the desiccant packets (36) from the desiccant holding area (42) and insertion of the desiccant packets (36) into the desiccant holding area (42) while the swab (14) is in the swab isolation area (41) of the desiccant chamber (32);
wherein the housing is configured to act both as a handle for the specimen collection portion during specimen collection and to act as a transport container during specimen shipping; and
wherein the desiccant chamber is configured to dry a specimen collected on the specimen collection portion during storage and shipment of the collected specimen, the desiccant chamber allowing the desiccant to be removed and new desiccant inserted;
wherein the swab (14) is positioned between desiccant packets (36).

2. The apparatus as claimed in claim 1 further comprising retaining structures (38)(40) in the desiccant chamber (32) the retaining structures (38)(40) configured to hold the desiccant packets (36) in the desiccant holding area (42) and away from contact with the swab (14).

3. The apparatus as claimed in claim 2 wherein the retaining structures (38) are rigid flanges (38) extending from opposed sidewalls of the desiccant chamber (32).

4. The apparatus as claimed in claim 1 wherein the desiccant packets contains a quantity of desiccant that is sized to correspond to a user selected swab drying time interval.

5. The apparatus as claimed in claim 2 wherein the retaining structures (40) are flexible retainers (40) extending downwardly from a top wall of the desiccant chamber.

6. The apparatus as claimed in claim 1 further comprising a reagent vial holder connected to the exterior of the desiccant chamber, the holder having a cylindrical shape and extending upwardly from a shoulder area of the chamber, the chamber shoulder area extending outwardly from the connection of said neck to said desiccant chamber and further comprising a vial for insertion into the reagent vial holder the vial configured to hold a known volume of a reagent for application to the swab.

7. The apparatus as claimed in claim 6 comprising a vial cap holder extending from the outside of the desiccant chamber.

8. The apparatus as claimed in claim 6 wherein the reagent vial holder is a "T" shape projection extending from the desiccant chamber resealable cover (34) and the vial comprises a "T" shaped void registrable with the "T" shape projection.

9. The apparatus as claimed in claim 8 wherein the vial holder is a "C-shaped" clamp having friction fit with said vial.

10. A method of specimen collection comprising the steps of:
a. providing a specimen collector device comprising
a-1 at least two specimen collectors extending from a first side of a container closure, each of the specimen collectors comprising:
(i) a specimen collection swab, the swab connected to a shaft having a first shaft end with the specimen collection swab thereon and a second shaft end connected to the container closure, the closure having first and second opposed sides of a central member, the specimen collector swabs being configured to simultaneously contact a specimen to collect a specimen sample on the swab, the specimen collector swabs being positioned to not contact the other specimen collector swab;
(ii) a generally rigid tube mounted coaxially on the shaft, the tube having a first end connected to a first stopper structure and a second end terminating at a shaft break-point on said shaft said shaft-break point being positioned along said shaft at a location sufficiently spaced from said swab to permit the entire swab to be separated from the shaft when the shaft is broken at said shaft break-point; and
a-2 a housing for holding and drying and transporting the specimen collection swabs, the housing comprising:
(i) a desiccant chamber;
(ii) a neck extending from the desiccant chamber, the neck having an opening providing communication through the neck and into the desiccant chamber for passage of the swab from the neck and into the desiccant chamber;
(iii) a swab isolation area in the desiccant chamber, the swab isolation area aligned with the neck to receive the shaft mounted swab from the neck and to position the swab in the swab isolation area of the desiccant chamber;
(iv) a desiccant holding area on at least two sidewalls of the desiccant chamber of the swab isolation area, the desiccant holding area configured to retain a desiccant packet therein to absorb moisture from a specimen collected on the swab; and
(v) an openable and closeable bottom on the desiccant chamber configured to permit removal of the desiccant packets from the desiccant holding area and insertion of the desiccant packets into the
(vi) desiccant holding area while the swab is in the swab isolation area of the desiccant chamber;
b. applying both swabs to a specimen;
c. collecting a sample of the specimen onto both swabs;
d. inserting simultaneously both swabs into the neck of the housing to place the swabs in the swab isolation area of the desiccant chamber;
e. aligning a first indicator located on the central member of the closure with a second indicator located on the holder, wherein both swabs are positioned within the desiccant holding area in a position that provides uniform separation between each of the swabs and the desiccant packets; and
f. drying both swabs simultaneously within the desiccant chamber.

11. The method of claim 10 wherein the first swab is applied to the specimen and the second swab is simultaneously applied to an area adjacent the specimen but which does not include the specimen to simultaneously obtain a sample of the specimen and a sample of the area adjacent the specimen.

12. The method of claim 11 further comprising the steps of:
analyzing the first swab sample of the specimen for data regarding the specimen to provide specimen data;
analyzing the second swab sample of the area immediately adjacent the specimen for data regarding the area, to provide area data; and
using the area data as a control for comparison to the specimen data to interpret the specimen data.

13. The method of claim 10 further comprising the steps of:
providing a flexible desiccant retainer to separate the desiccant holding area from the swab isolation area;
inserting a desiccant packet into the desiccant holding area; and
permitting the flexible desiccant retainer to move toward or away from the swab isolation area to allow accommodation of the volume of the inserted flexible desiccant packet.

14. The method of claim 10, wherein the steps of applying the swabs to a specimen and collecting a sample of the specimen onto both swabs are performed sequentially and not simultaneously.

15. The method of claim 10 further comprising the step of applying a solution to one of said at least two swabs prior to the step of applying the swabs to a specimen.

## Patentansprüche

1. Apparatur (10) zum Nehmen, Trocknen und Transportieren einer Probe, umfassend:
a. einen Probennehmer (12), umfassend:
einen Probennahmetupfer (14), wobei der Tupfer mit einem Schaft (16) verbunden ist, der ein erstes Schaftende mit dem Probennahmetupfer (14) daran und ein zweites, mit einem Verschluss (18) verbundenes Schaftende aufweist, wobei der Verschluss eine erste (22a) und eine zweite (22b) Anschlagkonstruktion aufweist, die von gegenüberliegenden Seiten eines zentralen Bauteils (20) ausgehen, wobei die erste Anschlagkonstruktion (22a) mit dem Schaft (16) verbunden ist; und
ein Abbrechröhrchen (24), das koaxial am Schaft (16) montiert ist, wobei das Abbrechröhrchen ein erstes Ende, das mit der ersten Anschlagkonstruktion (22a) verbunden ist, und ein zweites Ende, das an einem Schaft-Brechpunkt (27) am Schaft (16) endet, aufweist, wobei der Schaft-Brechpunkt (27) entlang des Schafts (16) an einer Stelle positioniert ist, die ausreichend von dem Tupfer (14) beabstandet ist, damit der ganze Tupfer (14) vom Schaft (16) abgetrennt werden kann, wenn der Schaft-Brechpunkt (27) gegen das Abbrechröhrchen (24) gedrückt wird;
b. ein Gehäuse, umfassend:
eine Trockenmittelkammer (32);
einen Hals (26), der von der Trockenmittelkammer (32) ausgeht, wobei der Hals eine Öffnung aufweist, die eine Verbindung durch den Hals und in die Trockenmittelkammer (32) herstellt, durch die der Tupfer aus dem Hals in die Trockenmittelkammer gelangen kann;
einen Tupferisolationsbereich (41) in der Trockenmittelkammer (32), wobei der Tupferisolationsbereich (41) derart nach dem Hals ausgerichtet ist, dass er den am Schaft montierten Tupfer aus dem Hals aufnimmt und den Tupfer in dem Tupferisolationsbereich (41) der Trockenmittelkammer (32) positioniert;
einen Trockenmittel-Haltebereich (42) an mindestens zwei Seiten des Tupferisolationsbereiches (41), wobei der Trockenmittel-Haltebereich (42) so ausgebildet ist, dass er Trockenmittelpäckchen (36) in sich hält, die Feuchtigkeit aus einer mit dem Tupfer (14) genommen Probe absorbieren; und
einen öffenbaren und verschließbaren Boden (34) an der Trockenmittelkammer, der so ausgebildet ist, dass er das Entfernen der Trockenmittelpäckchen (36) aus dem Trockenmittel-Haltebereich (42) und das Einbringen der Trockenmittelpäckchen (36) in den Trockenmittel-Haltebereich (42) ermöglicht, während sich der Tupfer (14) im Tupferisolationsbereich (41) der Trockenmittelkammer (32) befindet;
wobei das Gehäuse so ausgebildet ist, dass es sowohl als ein Griff für den Probennahmeabschnitt während der Probennahme als auch als ein Transportbehälter während des Versands der Proben dient; und
wobei die Trockenmittelkammer so ausgebildet ist, dass sie eine am Probennahmeabschnitt genommene Probe während der Lagerung und des Versands der genommen Probe trocknet, wobei es die Trockenmittelkammer ermöglicht, dass das Trockenmittel entfernt und neues Trockenmittel eingebracht werden kann;
wobei der Tupfer (14) zwischen den Trockenmittelpäckchen (36) positioniert ist.

2. Apparatur nach Anspruch 1, ferner umfassend Haltekonstruktionen (38)(40) in der Trockenmittelkammer (32), wobei die Haltekonstruktionen (38)(40) so ausgebildet sind, dass sie die Trockenmittelpäckchen (36) im Trockenmittel-Haltebereich (42) und ohne Kontakt mit dem Tupfer (14) halten.

3. Apparatur nach Anspruch 2, wobei die Haltekonstruktionen (38) starre Ansätze (38) sind, die von gegenüberliegenden Seitenwänden der Trockenmittelkammer (32) ausgehen.

4. Apparatur nach Anspruch 1, wobei die Trockenmittelpäckchen eine Menge an Trockenmittel enthalten, die so bemessen ist, dass sie einem vom Nutzer gewählten Tupfer-Trocknungszeitintervall entsprechen.

5. Apparatur nach Anspruch 2, wobei die Haltekonstruktionen (40) flexible Halterungen (40) sind, die ausgehend von der oberen Wandung der Trockenmittelkammer nach unten verlaufen.

6. Apparatur nach Anspruch 1, ferner umfassend eine Reagensphiolenhalterung, die mit der Außenseite der Trockenmittelkammer verbunden ist, wobei die Halterung zylindrisch ist und ausgehend von einem Schulterbereich der Kammer nach oben verläuft, wobei der Kammerschulterbereich ausgehend von der Verbindung des Halses mit der Trockenmittelkammer nach außen verläuft, und ferner umfassend eine Phiole zum Einsetzen in die Reagensphiolenhalterung, wobei die Phiole so ausgebildet ist, dass sie ein bekanntes Volumen eines Reagens zum Aufbringen auf den Tupfer hält.

7. Apparatur nach Anspruch 6, umfassend eine Phiolendeckelhalterung, die von der Außenseite der Trockenmittelkammer ausgeht.

8. Apparatur nach Anspruch 6, wobei die Reagensphiolenhalterung ein "T"-förmiger Vorsprung ist, der von der wiederverschließbaren Abdeckung der Trockenmittelkammer (34) ausgeht, und wobei die Phiole einen "T"-förmigen Hohlraum umfasst, der mit dem "T"-förmigen Vorsprung zusammenpasst.

9. Apparatur nach Anspruch 8, wobei die Phiolenhalterung eine "C-förmige" Klemme ist, die reibschlüssig an die Phiole passt.

10. Verfahren zur Probennahme, umfassend die Schritte:
a. Bereitstellen einer Probennehmervorrichtung, umfassend
a-1 mindestens zwei Probennehmer, die von einer ersten Seite eines Behälterverschlusses ausgehen, wobei jeder Probennehmer umfasst:
(i) einen Probennahmetupfer, wobei der Tupfer mit einem Schaft verbunden ist, der ein erstes Schaftende mit dem Probennahmetupfer daran und ein zweites, mit dem Behälterverschluss verbundenes Schaftende aufweist, wobei der Verschluss eine erste und eine zweite, gegenüberliegende Seite eines zentralen Bauteils aufweist, wobei die Probennehmertupfer so ausgebildet sind, dass sie gleichzeitig eine Probe kontaktieren und so eine Probe auf den Tupfer nehmen, wobei die Probennehmertupfer so positioniert sind, dass sie den anderen Probennehmertupfer nicht kontaktieren;
(ii) ein in der Regel starres Röhrchen, das koaxial am Schaft montiert ist, wobei das Röhrchen ein erstes Ende, das mit einer ersten Anschlagkonstruktion verbunden ist, und ein zweites Ende, das an einem Schaft-Brechpunkt am Schaft endet, aufweist, wobei der Schaft-Brechpunkt entlang des Schafts an einer Stelle positioniert ist, die ausreichend von dem Tupfer beabstandet ist, damit der ganze Tupfer vom Schaft abgetrennt werden kann, wenn der Schaft am Schaft-Brechpunkt abgebrochen wird; und
a-2 ein Gehäuse zum Halten und Trocknen und Transportieren der Probennahmetupfer, wobei das Gehäuse umfasst:
(i) eine Trockenmittelkammer;
(ii) einen Hals, der von der Trockenmittelkammer ausgeht, wobei der Hals eine Öffnung aufweist, die eine Verbindung durch den Hals und in die Trockenmittelkammer herstellt, durch die der Tupfer aus dem Hals und in die Trockenmittelkammer gelangen kann;
(iii) einen Tupferisolationsbereich in der Trockenmittelkammer, wobei der Tupferisolationsbereich so nach dem Hals ausgerichtet ist, dass er den am Schaft montierten Tupfer aus dem Hals aufnimmt und den Tupfer in dem Tupferisolationsbereich der Trockenmittelkammer positioniert;
(iv) einen Trockenmittel-Haltebereich an mindestens zwei Seitenwänden der Trockenmittelkammer des Tupferisolationsbereichs, wobei der Trockenmittel-Haltebereich so ausgebildet ist, dass er ein Trockenmittelpäckchen in sich hält, das Feuchtigkeit aus einer auf den Tupfer genommenen Probe absorbiert; und
(v) einen öffenbaren und verschließbaren Boden an der Trockenmittelkammer, der so ausgebildet ist, dass er das Entfernen der Trockenmittelpäckchen aus dem Trockenmittel-Haltebereich und das Einbringen der Trockenmittelpäckchen in den
(vi) Trockenmittel-Haltebereich ermöglicht, während sich der Tupfer im Tupferisolationsbereich der Trockenmittelkammer befindet;
b. Aufbringen einer Probe auf beide Tupfer;
c. Nehmen einer Stichprobe der Probe auf beide Tupfer;
d. gleichzeitiges Einbringen beider Tupfer in den Hals des Gehäuses zum Platzieren der Tupfer in dem Tupferisolationsbereich der Trockenmittelkammer;
e. Ausrichten eines ersten Anzeigers, der sich am zentralen Bauteil des Verschlusses befindet, nach einem zweiten Anzeiger, der sich an der Halterung befindet, wobei beide Tupfer in dem Trockenmittel-Haltebereich in einer Position positioniert sind, die für eine einheitliche Trennung zwischen jedem der Tupfer und der Trockenmittelpäckchen sorgt; und
f. Trocknen beider Tupfer gleichzeitig in der Trockenmittelkammer.

11. Verfahren nach Anspruch 10, wobei der erste Tupfer auf die Probe aufgebracht wird und der zweite Tupfer gleichzeitig auf einen Bereich neben der Probe, der die Probe jedoch nicht umfasst, aufgebracht wird, wodurch gleichzeitig eine Stichprobe von der Probe und eine Stichprobe vom Bereich neben der Probe genommen werden.

12. Verfahren nach Anspruch 11, ferner umfassend die Schritte:
Analysieren der ersten Tupferstichprobe von der Probe auf Daten bezüglich der Probe zur Bereitstellung von Probendaten;
Analysieren der zweiten Tupferstichprobe des Bereiches unmittelbar neben der Probe auf Daten bezüglich des Bereiches zur Bereitstellung von Bereichsdaten; und
Verwendung der Bereichsdaten als eine Kontrolle zum Vergleich mit den Probendaten zum Auslegen der Probendaten.

13. Verfahren nach Anspruch 10, ferner umfassend die Schritte:
Bereitstellen eines flexiblen Trockenmittelhalters zum Abtrennen des Trockenmittel-Haltebereiches vom Tupferisolationsbereich;
Einbringen eines Trockenmittelpäckchens in den Trockenmittel-Haltebereich; und
Ermöglichen, dass sich der flexible Trockenmittelhalter zum Tupferisolationsbereich hin oder von diesem weg bewegen kann, damit das Volumen des eingebrachten flexiblen Trockenmittelpäckchens angeglichen werden kann.

14. Verfahren nach Anspruch 10, wobei die Schritte Aufbringen der Tupfer auf eine Probe und Nehmen einer Stichprobe der Probe auf beide Tupfer nacheinander und nicht gleichzeitig ausgeführt werden.

15. Verfahren nach Anspruch 10, ferner umfassend den Schritt Aufbringen einer Lösung auf einen der mindestens zwei Tupfer vor dem Schritt Aufbringen der Tupfer auf eine Probe.

## Revendications

1. Dispositif de séchage, de transport et de collection d'échantillon (10) comprenant:
un collecteur d'échantillon (12) comprenant :
un écouvillon de collection d'échantillon (14), ledit écouvillon étant connecté à un arbre (16) ayant une première extrémité d'arbre avec ledit écouvillon de collection d'échantillon (14) sur celui-ci et une seconde extrémité d'arbre connectée à une fermeture (18), ladite fermeture ayant une première (22a) et une seconde (22b) structures d'arrêt s'étendant à partir de côtés opposés d'un élément central (20), ladite première structure d'arrêt (22a) étant connectée à l'arbre (16) ; et
un tube détachable (24) monté coaxialement sur l'arbre (16), le tube détachable ayant une première extrémité connectée à ladite première structure d'arrêt (22a) et une seconde extrémité se terminant en un point de rupture d'arbre (27) sur ledit arbre (16), ledit point de rupture d'arbre (27) étant positionné le long dudit arbre (16) en un emplacement suffisamment espacé dudit écouvillon (14) pour permettre à la totalité de l'écouvillon (14) d'être séparée de l'arbre (16) lorsque le point de rupture d'arbre (27) est appuyé contre le tube détachable (24) ;
b. un boîtier comprenant :
une chambre de dessiccation (32) ;
un goulot (26) s'étendant depuis la chambre de dessiccation (32), le goulot ayant une ouverture permettant la communication à travers le goulot et dans la chambre de dessiccation (32) afin de permettre le passage de l'écouvillon depuis le goulot et dans la chambre de dessiccation ;
une zone d'isolation d'écouvillon (41) dans la chambre de dessiccation (32), la zone d'isolation d'écouvillon (41) étant alignée avec le goulot pour recevoir l'écouvillon monté sur l'arbre depuis le goulot et pour positionner l'écouvillon dans la zone d'isolation d'écouvillon (41) de la chambre de dessiccation (32) ;
une zone de maintien de dessiccation (42) sur au moins deux côtés de la zone d'isolation d'écouvillon (41), la zone de maintien de dessiccation (42) étant configurée pour y retenir des sachets de dessiccation (36) afin d'absorber l'humidité d'un échantillon collecté sur l'écouvillon (14) ; et
un fond pouvant être ouvert et fermé (34) sur la chambre de dessiccation et configuré pour permettre le retrait des paquets de dessiccation (36) de la zone de maintien de dessiccation (42) et l'insertion des paquets de dessiccation (36) dans la zone de maintien de dessiccation (42), alors que l'écouvillon (14) se trouve dans la zone d'isolation d'écouvillon (41) de la chambre de dessiccation (32) ;
dans lequel le boîtier est configuré pour servir à la fois de poignée pour la partie de collection d'échantillon lors de la collecte d'échantillon et pour servir de conteneur de transport lors de l'expédition d'échantillon ; et
dans lequel la chambre de dessiccation est configurée pour sécher un échantillon collecté sur la partie de collecte d'échantillon pendant le stockage et l'expédition de l'échantillon collecté, la chambre de dessiccation permettant l'enlèvement du dessiccant et l'insertion d'un nouveau dessiccant ;
dans lequel l'écouvillon (14) est placé entre des paquets de dessiccation (36).

2. Dispositif selon la revendication 1, comprenant en outre des structures de retenue (38) (40) dans la chambre de dessiccation (32), les structures de retenue (38) (40) étant configurées pour maintenir les paquets de dessiccation (36) dans la zone de maintien de dessiccation (42) et loin du contact avec l'écouvillon (14).

3. Dispositif selon la revendication 2, dans lequel les structures de retenue (38) sont des brides rigides (38) s'étendant à partir de parois latérales opposées de la chambre de dessiccation (32).

4. Dispositif selon la revendication 1, dans lequel les paquets de dessiccation contiennent une quantité de dessiccant qui est dimensionnée pour correspondre à un intervalle de temps de séchage d'écouvillon sélectionné par l'utilisateur.

5. Dispositif selon la revendication 2, dans lequel les structures de retenue (40) sont des dispositifs de retenue flexibles (40) s'étendant vers le bas à partir d'une paroi supérieure de la chambre de dessiccation.

6. Dispositif selon la revendication 1, comprenant en outre un support de flacon de réactif connecté à l'extérieur de la chambre de dessiccation, le support ayant une forme cylindrique et s'étendant vers le haut depuis une zone d'épaule de la chambre, la zone d'épaule de chambre s'étendant vers l'extérieur depuis la connexion dudit goulot à ladite chambre de dessiccation et comprenant en outre un flacon destiné à être inséré dans le support de flacon de réactif, le flacon étant configuré pour maintenir un volume connu de réactif à appliquer sur l'écouvillon.

7. Dispositif selon la revendication 6, comprenant un support de bouchon de flacon s'étendant depuis l'extérieur de la chambre de dessiccation.

8. Dispositif selon la revendication 6, dans lequel le support de flacon de réactif est une projection en forme de "T" s'étendant depuis le couvercle refermable (34) de la chambre de dessiccation et le flacon comprend un vide en forme de "T" complémentaire à la projection en forme de "T".

9. Dispositif selon la revendication 8, dans lequel le support de flacon est un collier de serrage en "C" ayant un ajustement par friction avec ledit flacon.

10. Procédé de collecte d'échantillons comprenant les étapes consistant à :
a. fournir un dispositif de collection d'échantillon comprenant
a-1 au moins deux collecteurs d'échantillon s'étendant depuis un premier côté d'une fermeture de conteneur, chacun des collecteurs d'échantillon comprenant :
(i) un écouvillon de collection d'échantillon, l'écouvillon étant connecté à un arbre ayant une première extrémité d'arbre avec l'écouvillon de collection d'échantillon sur celui-ci et une seconde extrémité d'arbre connectée à la fermeture de conteneur, la fermeture ayant des premier et second côtés opposés à un élément central, les écouvillons de collection d'échantillon étant configurés pour entrer en contact simultanément avec un échantillon afin de collecter un prélèvement d'échantillon sur l'écouvillon, les écouvillons de collection d'échantillon étant positionnés pour ne pas être en contact avec l'autre écouvillon de collection d'échantillon ;
(ii) un tube globalement rigide monté coaxialement sur l'arbre, le tube ayant une première extrémité connectée à une première structure d'arrêt et une seconde extrémité se terminant à un point de rupture d'arbre sur ledit arbre, ledit point de rupture d'arbre étant positionné le long dudit arbre à un emplacement suffisamment espacé dudit écouvillon pour permettre à l'ensemble de l'écouvillon d'être séparé de l'arbre lorsque l'arbre est cassé audit point de rupture arbre ; et
a-2 un boîtier pour le maintien, le séchage et le transport des écouvillons de collection d'échantillon, le boîtier comprenant :
(i) une chambre de dessiccation ;
(ii) un goulot s'étendant depuis la chambre de dessiccation, le goulot ayant une ouverture permettant la communication à travers le goulot et dans la chambre de dessiccation pour le passage de l'écouvillon depuis le goulot et dans la chambre à dessiccation ;
(iii) une zone d'isolation d'écouvillon dans la chambre de dessiccation, la zone d'isolation d'écouvillon étant alignée avec le goulot pour recevoir l'écouvillon monté sur l'arbre et positionner l'écouvillon dans la zone d'isolation d'écouvillon de la chambre de dessiccation ;
(iv) une zone de maintien de dessiccation sur au moins deux parois latérales de la chambre de dessiccation de la zone d'isolation d'écouvillon, la zone de maintien de dessiccation étant configurée pour retenir un sachet de dessiccation afin d'absorber l'humidité d'un échantillon collecté sur l'écouvillon ; et
(v) un fond pouvant être ouvert et fermé sur la chambre de dessiccation, configuré pour permettre le retrait des paquets de dessiccation de la zone de maintien de dessiccation et l'insertion des paquets de dessiccation dans la
(vi) zone de maintien de dessiccation pendant que l'écouvillon se trouve dans la zone d'isolation d'écouvillon de la chambre de dessiccation ;
b. appliquer les deux écouvillons sur un échantillon ;
c. collecter un prélèvement de l'échantillon sur les deux écouvillons ;
d. insérer simultanément les deux écouvillons dans le goulot du logement pour placer les écouvillons dans la zone d'isolation d'écouvillon de la chambre de dessiccation ;
e. aligner un premier indicateur situé sur l'élément central de la fermeture avec un second indicateur situé sur le support, dans lequel les deux écouvillons sont positionnés dans la zone de maintien de dessiccation dans une position qui assure une séparation uniforme entre chacun des écouvillons et les paquets de dessiccation ; et
f. sécher les deux écouvillons simultanément dans la chambre de dessiccation.

11. Procédé selon la revendication 10, dans lequel le premier écouvillon est appliqué sur l'échantillon et le second écouvillon est appliqué simultanément sur une zone adjacente à l'échantillon mais qui n'inclut pas l'échantillon pour obtenir simultanément un prélèvement de l'échantillon et un prélèvement de la zone adjacente à l'échantillon.

12. Procédé selon la revendication 11, comprenant en outre les étapes consistant à :
analyser le premier prélèvement d'écouvillon de l'échantillon pour obtenir des données concernant l'échantillon afin de fournir des données d'échantillon ;
analyser le second prélèvement d'échantillon de la zone immédiatement adjacente à l'échantillon pour obtenir des données concernant la zone, afin de fournir des données de zone ; et
utiliser les données de zone comme une commande de comparaison des données d'échantillon pour interpréter les données d'échantillon.

13. Procédé selon la revendication 10, comprenant en outre les étapes consistant à :
fournir un dispositif de retenue de dessiccation flexible pour séparer la zone de maintien de dessiccation de la zone d'isolation d'écouvillon ;
insérer un paquet de dessiccation dans la zone de maintien de dessiccation ; et
permettre au dispositif de retenue de dessiccation flexible de se déplacer vers ou hors de la zone d'isolation d'écouvillon pour permettre l'adaptation du volume du paquet de dessiccation flexible inséré.

14. Procédé selon la revendication 10, dans lequel les étapes consistant à appliquer les écouvillons sur un échantillon et à collecter un prélèvement de l'échantillon sur les deux écouvillons sont effectuées séquentiellement et non simultanément.

15. Procédé selon la revendication 10, comprenant en outre l'étape consistant à appliquer une solution à l'un desdits au moins deux écouvillons avant l'étape consistant à appliquer les écouvillons à un échantillon.
